# EUROPÄISCHE PATENTSCHRIFT

(11) **EP 1 565 197 B1**
(45) Veröffentlichungstag und Bekanntmachung des Hinweises auf die Patenterteilung: **13.10.2010**
(21) Anmeldenummer: 03775619.4
(22) Anmeldetag: 26.11.2003
(51) Int. Cl.: A61P 1/00, A61K 36/185

(54) **DIE VERWENDUNG VON PAPAYA-MUS ZUR BEHANDLUNG VON VERDAUUNGSSTÖRUNGEN**
USE OF PAPAYA PUREE FOR THE TREATMENT OF DIGESTIVE TROUBLES
UTILISATION D'UNE COMPOTE DE PAPAYE POUR LE TRAITEMENT DES TROUBLES DIGESTIFS

(30) Priorität: 26.11.2002 AT 17712002
(43) Veröffentlichungstag der Anmeldung: 24.08.2005
(73) Patentinhaber: Ji Kwang Inc., Hilo, Hawaii 96720 (US)
(72) Erfinder: Choi, Danette Vanessa, Mountain View, HI 96771 (US)
(74) Vertreter: Sonn & Partner Patentanwälte
(86) Internationale Anmeldenummer: PCT/IB2003/005476
(87) Internationale Veröffentlichungsnummer: WO 2004/047850

(56) Entgegenhaltungen:
- CN-A- 1 345 543
- DAWSON EMMA: "The medicinal properties of the papaya, Carica papaya L." INTERNET ARTICLE, [Online] 15. Mai 1998 (1998-05-15), Seiten 1-3, XP002271935 Gefunden im Internet: <URL:www.siu.edu/bl/leaflets/papaya.htm> [gefunden am 2004-02-18]
- PEREZ B ET AL: "Study on thermal processing of papaya pieces" ALIMENTARIA, Bd. 37, Nr. 310, März 2000 (2000-03), Seiten 101-104, XP009026308 ISSN: 0300-5755
- PATENT ABSTRACTS OF JAPAN vol. 1996, no. 07, 31. Juli 1996 (1996-07-31) & JP 08 056562 A (NAKAYAMA AKIRA), 5. März 1996 (1996-03-05)
- LOPEZ HERNANDEZ, J. A. ET AL: "[Papaya jam and 'dulce'.]" MISCELANEA, UNIVERSIDAD NACIONAL DE TUCUMAN FACULTAD DE AGRONOMIA Y ZOOTECNIA, (1976), NO. 55, 1-13, 7 REF., Bd. 55, 1976, Seiten 1-13, XP001179585 Fac. de Agron. y Zootecnia, Univ. Nat. de Tucuman, Tucuman, Argentina
- M. GAMAL ABD EL-AL ET AL: "Application of Microwave Energy in the Heat Treatment of Fruit Juices, Concentrates, and Pulps" PROCESSING, Oktober 1994 (1994-10), Seiten 307-312, XP008025211
- DATABASE MEDLINE [Online] US NATIONAL LIBRARY OF MEDICINE (NLM), BETHESDA, MD, US; November 1999 (1999-11) STARLEY I F ET AL: "The treatment of paediatric burns using topical papaya." Database accession no. NLM10563690 XP002271936 & BURNS: JOURNAL OF THE INTERNATIONAL SOCIETY FOR BURN INJURIES. ENGLAND NOV 1999, Bd. 25, Nr. 7, November 1999 (1999-11), Seiten 636-639, ISSN: 0305-4179

## Beschreibung

Die Erfindung betrifft die Verwendung eines Mus-Präparates aus *Carica papaya*-Früchten zur Herstellung eines Mittels zur Behandlung von Verdauungsstörungen.

*Carica papaya* (Melonenbaum) gehört zur Familie *Caricaceae* der *Violales* und produziert große, saftige und wohlschmeckende Früchte (Papayas).

Die Papaya stammt aus tropischen Regionen, wo sie auch kultiviert wurde. Großangelegte Plantagen findet man in Ceylon, Pakistan, Indien, Australien, Ostafrika und Brasilien.
In Mexiko und Zentralamerika findet man ebenso viele Plantagen, die jedoch wesentlich kleiner sind. Der Baum wird bis zu sechs Meter hoch, die Früchte können ein Gewicht von bis zu 7 kg erreichen.

In traditionellen medizinischen Kulturen wird Papaya (Schale, Fruchtfleisch, Samen; selten auch Blätter und Latex) vor allem bei Asthma, Parasitosen, Wundheilungsstörungen sowie gastrointestinalen Problemen wie Diarrhoe oder Obstipation eingesetzt. Die Inhaltsstoffe stimulieren und regulieren die Verdauungstätigkeit, mildern eine Übersäuerung des Magens, vermindern eine zu starke Gasbildung und unterstützen die Aufspaltung von Proteinen.

Über mögliche Heilwirkungen wurde erstmals schriftlich vom Spanier Oviedo berichtet (1526). Dr. Mario Rojas Alba, Präsident des mexikanischen Institutes für Traditionelle Medizin beschäftigt sich seit 1996 intensiv mit der heilsamen Wirkung dieser Frucht.

Bisher wurden sechs verschiedene Enzyme isoliert:
- Papain
- Chymopapain A und B
- Lysozyme
- Lipase
- Glutamin-Cyclopherase
- Kallose
Weiters ist die Papaya sehr reich an:
- Pektin
- Vitamin A,B,C
- Essentiellen Fettsäuren
- Bioflavonoiden
- Kalium
- Kalzium
- Magnesium
- Phosphotiden
- Peptiden
- Aminosäuren (z.B. Arginin)

Das Glykosid Carpain soll einen herzmuskelstärkenden Effekt aufweisen.

Neben der Verwendung als Lebensmittel werden die Früchte von Ca*rica papaya* zur Herstellung des proteolytischen Enzyms Papain verwendet.

Papain wurde zur Verhinderung von Brandwundeninfektionen, zur Defibrination von Wunden, zur Behandlung von Insektenstichen, zur Behandlung von Ödemen, entzündlichen Prozessen und zur Beschleunigung der Wundheilung sowie - in geringen Dosierungen - bei Magenverstimmungen verwendet. Papayas werden weiters als laxativ und erfrischend beschrieben.

Papain (Papaya peptidase I, EC 3.4.22.2) wird dabei aus dem Milchsaft (Latex) unreifer Papayas gewonnen, wobei dieser Milchsaft eingetrocknet und pulverisiert wird.

Dawson (www.siu.edu/-ebl/leaflets/papaya.htm) gibt einen allgemeinen Überblick über die medizinische Verwendung von Teilen der Papayapflanze. Dabei wird die Verwendung von Wurzeln und Blättern bei Harnausscheidungsproblemen, von Blättern und Samen bei Wurmbefall und von der Frucht bei Krankheiten betreffend die Galle eingesetzt. Aufgrund des in der Papaya vorkommenden proteolytischen Enzyms Papain werden enzymreiche Teile der Pflanze weiters zur Unterstützung der Verdauung eingesetzt.

In Perez et al. (Alimentaria 37 (310), 101-104, 2000) wurden Möglichkeiten zur Sterilisation von in Dosen abgefüllten Papayastücken in einem Zuckersaft untersucht. Das darin geoffenbarte Verfahren hat die Optimierung der Sterilisationsbedingungen sowohl in Hinblick auf die sensorischen Eigenschaften als auch in Hinblick auf die Produktionskosten.

JP 08 056562 A offenbart ein Verfahren zur Herstellung von in Pflaumensaft befindlichen Papayastücken. Dabei wird die Papayafrucht zwei bis drei Tage vor der Reifung in Stücke geschnitten und geschält. Anschließend werden die Papayastücke in Pflaumensaft, der 25-35% Zucker enthält, für 10 Minuten gekocht.

CN 1 345 543 A offenbart gemäß der Zusammenfassung ein Verfahren zur Herstellung von konservierter Papayafrucht, welche einen geringen Anteil an Fructose aufweist. Dabei wird die Frucht zunächst in einer wässrigen Lösung, die mehrere Salze enthält, behandelt um die Farbe der Frucht zu erhalten und die in der Papaya enthaltenen Gerbstoffe zu entfernen. Anschließend wird die Frucht gekocht, in einer Zuckerlösung eingeweicht, überschüssiger Zucker entfernt, getrocknet und verpackt.

Lopez et al. (Miscelanea, Universidad Nacional de Tucuman Facultad de Agronomia y zootecnia 55, 1-13, 1976) offenbart ein Verfahren zur Herstellung von Papaya-Marmelade und Papaya-Süßigkeiten. Im Zuge der Herstellung dieser Papayaprodukte wird aus der reifen Frucht eine Pulpe hergestellt, die nach pH-Korrektur für 2 Minuten bei 96°C erhitzt wird. Das dadurch gewonnene Zwischenprodukt wird anschließend mit Hilfe eines Filters von nicht erwünschten Bestandteilen beispielsweise Samen oder Fasern gereinigt. Diese Pulpe wird schlussendlich zur Herstellung von Marmeladen und Süßigkeiten nach dem Stand der Technik bekannten Verfahren eingesetzt.

Gamal et al. (Processing 1994, 307-312) offenbart ein alternatives Verfahren zur Erhitzung von Fruchtsäften, Konzentraten und Pulpen. Die Autoren verglichen in ihrer Studie die Erhitzungszeiten, die für einen erwünschten Effekt in Fruchtzubereitungen nötig sind und fanden heraus, dass die Verwendung von Mikrowellen die Erhitzungszeiten gegenüber herkömmlichen Methoden stark reduzierten.

Starley et al. (J. Int. Soc. Burn Inj. 25, 636-639, 1999) offenbart die Verwendung von Papaya zu medizinischen Zwecken. Dabei wurde festgestellt, dass sich Papaya zum Lösen nekrotischer Gewebe und zur Prävention von Brandwundeninfektionen eignet und im Zuge dessen eine geeignete Wunde erzeugt, um eine Hauttransplantation durchzuführen.

Die Aufgabe der vorliegenden Erfindung besteht darin, ein Mittel zur Behandlung von Verdauungsstörungen bzw. ein Verdauung-regulierendes Mittel zur Verfügung zu stellen.

Die vorliegende Erfindung betrifft daher die Verwendung eines Mus-Präparates aus Carica papaya-Früchten, erhältlich nach einem Verfahren, gekennzeichnet durch die folgenden Schritte:
- Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
- Abkühlen der gekochten Früchte oder zerkleinerten Früchte über einen Zeitraum von zumindest 30 min unter Sauerstoff-haltiger Atmosphäre,
- gegebenenfalls Zerkleinern, Mischen und Passieren der abgekühlten Früchte oder zerkleinerten Früchte bis zum Erhalt eines homogenen Muses zur Herstellung eines Mittels zur Behandlung von Verdauungsstörungen.

Ein derart hergestelltes Mus, bei welchem die Kochzeit und die Abkühlzeit entsprechend berücksichtigt wurden, erwies sich überraschenderweise als besonders geeignet zur Behandlung von Verdauungsstörungen; eine Eigenschaft, die bei Nichteinhaltung dieser Koch- und Abkühlzeit nicht oder nur in äußerst geringem Ausmaß eintritt.

Die Mindestdauer von 30 min bezieht sich auf Kochbedingungen bei Normaldruck, die Mindestkochzeit reduziert sich aber entsprechend, wenn das Kochgut unter Druck gekocht wird.

Das erfindungsgemäße Kochen der Früchte erfolgt - abhängig vom Wassergehalt der eingesetzten Früchte - vorzugsweise mit dem mindestens zweifachen Volumen einer wässerigen Lösung, vorzugsweise mit dem etwa vierfachen Volumen. Diese Ausführungsform ist besonders geeignet, wenn die ganzen Früchte gekocht werden oder bei zerkleinerten Früchten mit niedrigem Wassergehalt. Auch hierbei gilt, dass beim Kochen in einem geschlossenen Gefäß (unter Druck) sich die notwendige Wassermenge auf einen Bruchteil reduziert, da ansonsten bei mindestens zweifacher Wassermenge das Endprodukt zu verdünnt wäre. Das Kochen wird üblicherweise unter Zusatz von herkömmlichem Leitungswasser ohne Zusätze durchgeführt. Vorzugsweise wird das Kochen für mindestens 2 h, vorzugsweise für mindestens 3 h, insbesondere für mindestens 5 h durchgeführt. Der Abkühlvorgang wird z.B. dadurch erzielt, dass keine Wärmeenergie-Zuführung mehr erfolgt oder aber indem die Gefäße, in denen die Früchte gekocht werden, vom Kochplatz auf einen Platz mit Raumtemperatur gebracht werden. Dieser Abkühlprozess ist wie erwähnt ebenfalls sehr wichtig für die Generierung der Verdauung-regulierenden Eigenschaften des erfindungsgemäßen Muses und sollte für mindestens 30 min, vorzugsweise für mindestens 5 h, noch bevorzugter für mindestens 6 h (bzw. für 5 h bis 7 h) durchgeführt werden; jedoch sind auch Abkühlungszeiten von 10 h und mehr möglich. Beim Abkühlprozess ist Sauerstoffzufuhr wichtig, vorzugsweise wird unter Luftzufuhr gearbeitet.

Vorzugsweise kann bei der Herstellung des Muses, insbesondere im Rahmen des Zerkleinerungs-, Mischungs- und Passierungsschritts, Zitronensäure zur Verbesserung der Haltbarkeit zugegeben werden. Ebenfalls bevorzugt ist eine anschließende Pasteurisierung des erhaltenen Muses bei üblichen lebensmitteltechnologischen Bedingungen.

Um ein Endprodukt zu erhalten, das in seiner Konsistenz und Form noch appetitlicher aussieht und noch besser eingenommen werden kann, können die Früchte vor dem Kochprozess geschält und entkernt werden. Bei dieser Gelegenheit können die Früchte auch gleich vor dem Kochprozess zerkleinert werden, z.B. durch Siebung.

Die besten Resultate werden mit *Carica papaya*-Früchten erzielt, die halbreif bis reif sind. Der Reifezustand der Papayas lässt sich anhand der Farbe der Schale definieren: Unreife Früchte haben eine 100% grüne Schale, halbreife sind 50-75% gelb, reife Früchte sind 80-100% gelb.

Das Mus-Präparat aus *Carica papaya*-Früchten, welches mit dem erfindungsgemäßen Verfahren hergestellt werden kann, wird zur Herstellung eines Mittels zur Behandlung von Verdauungsstörungen verwendet. Wie oben erwähnt ist das erfindungsgemäß hergestellte Produkt überraschenderweise zur Behandlung von Verdauungsstörungen, insbesondere zur Behandlung von chronischer Obstipation, von Blähung und von Reizdarmsyndrom besonders gut geeignet, dies ist umso überraschender, als diese Eignung nicht vorrangig nur in der Natur von *Carica papaya*-Früchten liegt, sondern in der speziellen Zubereitung des erfindungsgemäßen Muses.

Das erfindungsgemäß hergestellte Mus kann auch im nicht-therapeutischen Bereich verwendet werden, z.B. zur Verbesserung des Verdauungsprozesses in an sich gesunden Menschen ohne bestehende Verdauungsstörungen, z.B. als Lebensmittel-Zusatz oder -Ergänzung.

Das gemäß dem erfindungsgemäßen Verfahren erhältliche Mus-Präparat aus *Carica papaya*-Früchten selbst, ist wie oben erwähnt durch die besondere Art der Herstellung, insbesondere die Koch- und Abkühlzeiten, besonders wirksam und daher besonders geeignet für die erfindungsgemäß geschilderten Indikationen.

Der Wassergehalt des erfindungsgemäßen Präparates kann dabei bevorzugterweise von 9 bis 90 % betragen, wobei die jeweilige Indikation von besonderer Bedeutung bei der Einstellung des Wassergehaltes ist. Als besonders bevorzugt hat sich dabei ein Wassergehalt von 60 bis 85 % herausgestellt, insbesondere von 70 bis 80 %, da dieser Wassergehalt nicht nur eine besondere Wirksamkeit, sondern auch wesentliche Vorteile bei der Verabreichung mit sich bringt:

So haben z.B. baumreif gepflückte Früchte einen Wassergehalt von etwa 88 % und einen Zuckergehalt von 8 bis 12 %. Wenn man daraus nun ein 1:1 Produkt produzieren würde, müsste beispielsweise bei den nachfolgend geschilderten Dosisangaben zur Verbesserung bei Obstipation die doppelte Dosis verabreicht werden, also statt zweimal zwei Esslöffel oder zweimal 20 ml eben zweimal vier Esslöffel oder zweimal 40 ml. Bei einer Konzentration von etwa 2:1 im Hinblick auf baumreif gepflückte Früchte, eine Konzentration die besonders bevorzugt ist, resultiert ein Endprodukt mit einem Wassergehalt von etwa 77 % und einem Zuckergehalt von 16 bis 24 %.

Gerade der Zuckergehalt ist aber auch ein wesentlicher Produktparameter des erfindungsgemäßen Muses. Dieser Zuckergehalt beträgt vorzugsweise 5 bis 40 %, besonders bevorzugt von 10 bis 30 %, insbesondere von 12 bis 26 %. Besonders eingestellt werden muss der Zuckergehalt selbstverständlich bei der Behandlung von Diabetes-Patienten bzw. Diabetes gefährdeten Patienten, etwa in der Geriatrie.

Besonders überraschend ist die Wirksamkeit des erfindungsgemäß erhältlichen Präparates insbesondere aufgrund der Tatsache, dass das Herstellungsverfahren einen lang andauernden Kochvorgang miteinschließt. Dies ist vor allem im Hinblick auf die Wirkung bei der Verbesserung der Verdauungsprozesse, insbesondere bei chronischer Obstipation, bemerkenswert, weil alle bekannten Publikationen betreffend eine positive verdauungsfördernde Wirkung von Papaya-Zubereitungen auf die allgemein verdauungsfördernde Wirkung des Enzyms Papain gestützt sind bzw. durch die Wirkung von Papain begründet werden. Papain wird jedoch bei Behandlungstemperaturen von über 85°C nachweislich gespalten und unwirksam, so dass die erfindungsgemäße Herstellungsweise diesem Stand der Technik völlig zuwider läuft und entgegen steht. Umso überraschender waren daher die im Beispielteil gezeigten Ergebnisse der klinischen Studien in denen die Wirkung unter anderem bei chronischer Obstipation und chronischer Diarrhoe gezeigt worden ist, trotz offenkundiger Papain-spaltender Herstellungsweise.

Die Dosierung kann im Einzelfall sehr stark von dem jeweiligen Krankheitsbild abhängen, das man behandeln will. So erfordert beispielsweise die Behandlung chronischer Diarrhoe eine wesentlich niedrigere Dosierung als die Behandlung von Obstipation, so dass es zu einer ausreichenden Wirksamkeit kommt, weshalb auch der Wassergehalt z.B. bei der Behandlung von chronischer Obstipation eher im niedrigen Bereich eingestellt wird (beispielsweise zwischen 70 und 85 %), wohingegen bei Mitteln, die zur Diarrhoe-Behandlung eingesetzt werden, ein höherer Wassergehalt bzw. auch eine niedrigere Dosierung angewendet werden.

Die Einstellung des Wassergehaltes kann in einfacher Weise im Rahmen des Kochvorganges erfolgen. Je nach Wassergehalt der eingesetzten Früchte kann beim erfindungsgemäßen Herstellungsverfahren ein Wasserzusatz erfolgen, oder auch nicht, wenn beispielsweise die Papayas bereits in zerkleinerter (pürierter, gesiebter, etc.) Form dem Kochvorgang unterzogen werden, kann ein Wasserzusatz vorzugsweise unterbleiben. Dies ist eine Herstellungsweise die sich insbesondere bei der Herstellung des erfindungsgemäßen Papaya-Muses vor Ort besonders bewährt hat, da diese Früchte bei der Ernte in der Regel einen höheren Wassergehalt aufweisen.

Die Erfindung wird anhand der nachfolgenden Beispiele, auf die sie selbstverständlich nicht eingeschränkt ist, näher erläutert.

### Beispiele

### 1. Herstellung des erfindungsgemäßen Muses aus Carica papaya-Früchten

100 kg Papaya-Früchte mit mindestens 20% Gelbanteil der Schale werden geschält und entkernt, in ein 500 1 Kochgefäß gefüllt und mit 300 1 Leitungswasser versetzt. Bei offenem Gefäß werden die Früchte mit Wasser anschließend 3 h lang gekocht und dann für 6 h bei Raumtemperatur offen auskühlen gelassen.

Nach dem Abkühlen wird dem Kochgut soviel Zitronensäure beigemengt, bis der pH-Wert auf etwa 3,8% reduziert wird (ca. 600 g).

Anschließend wird das abgekühlte Kochgut zu einem feinen Mus gemixt oder passiert, in 1 1-Gläser abgefüllt, verschlossen und pasteurisiert.

### 2. Herstellung des erfindungsgemäßen Muses in größeren Mengen

Bei der Erzeugung größerer Mengen hat sich, insbesondere wenn die Herstellung vor Ort (im Papaya-Herkunftsland) erfolgen soll, die folgende Vorverarbeitung bewährt:

Die Früchte werden gewaschen, anschließend maschinell von Schalen und Kernen befreit (bereits an dieser Stelle werden die Früchte durch ein Sieb getrieben um die Kerne zu eliminieren, damit kann das Pürieren nach dem Kochen entfallen). Dann wird Zitronensäure beigemengt, so dass der pH Wert auf 3,5 bis 5,0, insbesondere auf 3,8 bis 4,4 eingestellt wird und das Mus pasteurisiert und keimfrei in Tonnen abgefüllt. So kann es per Luft- oder Seefracht verschickt werden.

Wenn Vorprodukte aus der halbreif bis reifen Papaya einen höheren Wassergehalt, z.B. an die 88 %, als es die z.B. auf dem europäischen Markt erhältlichen Früchte haben, ist es gar nicht erforderlich, Wasser zum Kochen zuzugeben, insbesondere wenn die Zerkleinerung bereits vor dem Kochen vorgenommen worden ist. Um auf die produkttechnisch erwünschte Konsistenz und Konzentration zu kommen, kann sogar noch das Halbfertigfabrikat soweit durch den Kochvorgang konzentriert werden, dass der Wassergehalt auf etwa 77 % sinkt und der Zuckergehalt auf etwa 16 % steigt (dieser liegt bei unreif gepflückten Früchten bei etwa 5 % und bei-halbreif bis reif gepflückten Früchten bei etwa 8 bis 12 %). Praktisch wird dies in der Regel verwirklicht, indem 1 Ltr. "Rohmus" auf etwa ½ Ltr. "Fertigmus" konzentriert wird.

Nach dem Koch- und Abkühlungsvorgang, der essentiell für die Wirkung ist und unverändert bleibt, gibt es zwei bevorzugte Wege hin zur Verpackung:
A) Glasverpackung
   Dabei wird das Mus-Konzentrat (lt. Lebensmittelverordnung spricht man ab einer Konzentration von 2:1 von einem Konzentrat) in Glasflaschen gefüllt und anschließend pasteurisiert.
B) Schlauchbeutelverpackung oder StickPack-Verpackung Das Mus-Konzentrat wird in StickPacks portioniert (a 20 ml) abgefüllt, verschweißt und anschließend ebenfalls pasteurisiert oder gleich heiß aseptisch abgefüllt.

### 3. Studie zur Wirksamkeit des erfindungsgemäßen Papaya-Präparates bei geriatrischen Patienten mit chronischer Obstipation.

### 3.1. BESCHREIBUNG DES STUDIENPRÄPARATES

Die baumreif gepflückten Papayafrüchte werden geschält und entkernt und werden anschließend zwischen 30 min. und 5 Stunden mit dem mindestens 2-fachen Volumen einer wässrigen Lösung gekocht. Danach werden die gekochten Früchte über einen Zeitraum von mindestens 30 min. unter Sauerstoff-haltiger Atmosphäre abgekühlt, passiert und Zitronensäure zugesetzt. Für die Studie wurde das Präparat in Gläser abgefüllt und pasteurisiert, nach dem Öffnen wurden die Gläser im Kühlschrank aufbewahrt.

### 3.2. STUDIENDESIGN

In die Studie wurden 40 Patienten bzw. Bewohner eines Geriatriezentrums, die großteils immobil sind, aufgenommen. Alle hatten die ärztliche Diagnose einer chronischen Obstipation. Alle erhielten tgl. bis mindestens 3 mal wöchentlich Laxanzien. Mehr als 95 % erhielten Macrogol (Movicol®).

Ausgeschlossen waren Patienten bzw. Bewohner, die ein oder mehrere der aufgeführten Kriterien erfüllten:
- Bekanntes Malignom
- Stoma
- Blut im Stuhl
- Höhergradige Herzinsuffizienz
- Vorliegende Morphintherapie

Der Studienablauf gliederte sich in eine **Vorlaufperiode** von 19 Tagen, eine **Untersuchungsperiode** 1 von 35 Tagen, in denen 2 x tgl. das erfindungsgemäße Präparat verabreicht wurde (2 EL vor dem Frühstück mit etwas Wasser, 2 EL vor dem Mittagessen mit etwas Wasser),eine **Untersuchungsperiode 2** von 19 Tagen, in der das erfindungsgemäße Präparat auf eine morgendliche Verabreichung reduziert wurde (2 EL vor dem Frühstück mit etwas Wasser).

Während des gesamten Untersuchungszeitraumes wurde das Stuhlverhalten der Patienten äußerst genau auf speziellen Dokumentationsblättern notiert.

Als "escape"-Therapie, also wenn trotz Verabreichung des Studienpräparates kein Stuhlgang erfolgte, wurden am 3.Tag ohne Stuhlgang 2 Mikroklist® appliziert, am 4.Tag ohne Stuhlgang 1 Relaxyl®.

### 3.3. STUDIENVERLAUF

Es wurden 40 Patienten bzw. Bewohner in die Studie aufgenommen, die die oben genannten Kriterien erfüllten.

In der **Vorlaufphase** schieden 8 Patienten aus der Studie aus. Die Gründe waren fehlende Compliance, der Geschmack des erfindungsgemäßen Präparates wurde abgelehnt oder die Kontrolle des Stuhlganges war nicht ausreichend gegeben, da von diesen einige trotz eingeschränkter Mobilität manchmal selbständig die Toilette benutzten.

In der **Untersuchungsphase 1** zeigten 13 Patienten (von noch insgesamt 32) eine Verbesserung des Obstipationsgrades (Quotient aus Anzahl der Tage ohne Stuhl dividiert durch Anzahl der Tage mit Stuhl). 0 bedeutet täglicher Stuhlgang, 1 bedeutet 50% der Tage sind ohne Stuhlgang, etc ... 5 Patienten zeigten eine geringe Zunahme der stuhlfreien Tage im Vergleich zur Vorlaufphase, zeigten jedoch in der Untersuchungsphase 2 eine deutliche Besserung im Vergleich zur Vorlaufphase. 11 Patienten zeigten eine Verschlechterung des Obstipationsgrades. 4 Patienten wiesen genau den gleichen Obstipationsgrad wie in der Vorlaufphase auf - zeigten also auch keine Verschlechterung.
In der **Untersuchungsphase 2** wurden insgesamt noch 18 Patienten weiterbeobachtet. Von diesen zeigten 13 Patienten trotz Dosisreduktion eine weitere kontinuierliche Abnahme des Obstipationsquotienten. Bei 3 Patienten kam es im Vergleich zur Vorlaufphase zu einem progredienten Anstieg des Obstipationsquotienten (möglicherweise ist hier auch eine medikamentöse Interaktion zu berücksichtigen, da diese Patienten mehrere Psychopharmaka erhielten). Hier wäre noch eigens eine Untersuchung mit höherer Dosierung des Studienpräparates empfehlenswert. 1 Patient hatte während der gesamten Studiendauer täglich Stuhlgang. 1 Patient zeigte in der Untersuchungsphase 2 (Dosisreduktion) eine Verschlechterung im Vergleich zu Untersuchungsphase 1, war jedoch deutlich besser im Vergleich zur Vorlaufphase.

### 3.4. Zusammenfassung

In dieser an 40 Patienten bzw. Bewohnern einer geriatrischen Abteilung (Immobilität, Multimorbidität) durchgeführten prospektiven Studie mit einem so genannten Nahrungsergänzungsmittel, einer erfindungsgemäß zubereiteten Form des Fruchtfleisches der Papaya (=CARICOL^{®}) konnte ein signifikanter Effekt auf die Verbesserung der Stuhlgewohnheiten ohne Intervention mit Laxanzien nachgewiesen werden. Zu erwähnen ist der Verlauf des Obstipationsgrades bei einer Patientin mit PEG-Sonde, bei der das Präparat mit einer so genannten Alexanderspritze appliziert wurde (hier konnte die Dosierung am exaktesten eingehalten werden): Vorlaufphase 1.38, 1. Untersuchungsphase 0.4, 2. Untersuchungspahase 0.

### 4. Verwendung des erfindungsgemäßen Papaya-Muses zur Behandlung von chronischer Obstipation

Das erfindungsgemäße Papayamus reguliert bzw. verbessert den Verdauungsprozess, insbesondere bei Durchfall, Verstopfung (auch chronischer Verstopfung), Blähungen oder dem Reizdarmsyndrom.

Dazu werden am Morgen und zu Mittag jeweils 2 Esslöffel Papayamus zu den Mahlzeiten eingenommen. Die verdauungsregulierende Wirkung stellt sich in den meisten Fällen bereits am nächsten oder übernächsten Tag ein.

### 5. Studie zur Wirksamkeit des erfindungsgemäßen Papaya-Präparates bei geriatrischen Patienten mit chronischer Diarrhoe

### Studiendesign:

In die Anwendungsbeobachtung wurden 10 Patienten bzw. Bewohner der Pflegestation eines Pensionisten-Wohnheims, die großteils unter Diarrhoe leiden, aufgenommen.

Ausgeschlossen wurden Patienten bzw. Bewohner, die eine oder mehrere der angeführten Kriterien erfüllten:
- Bekanntes Malignom
- Stoma
- Blut im Stuhl

Nach einer **Vorlaufperiode** von 2 Wochen bei 4 Patienten und 3,5 Wochen bei 6 Patienten wurde den Patienten *CARICOL*^{®} (erfindungsgemäßes Papaya-Präparat)in zwei Therapiephasen verabreicht. In der **Phase 1** wurde *CARICOL*^{®} während 3,5 Wochen (bei 6 Patienten) und 5 Wochen (bei 4 Patienten) 2 mal täglich in der einfachen Konzentration verabreicht. Das Stuhlverhalten wurde genauestens dokumentiert.

Danach wurde die Therapie für drei Wochen ausgesetzt.

In der anschließenden **Phase 2** wurde die Verabreichung von *CARICOL*^{®} über 6 Wochen mit der doppelten Konzentration, ebenfalls 2 mal täglich fortgesetzt.

### Dosierung:

Phase 1: Einfache Konzentration von *CARICOL*^{®}*,* Tagesdosis 40 ml, das entspricht der Menge von 2x2 Esslöffel. Die Einnahme erfolgte jeweils kurz vor den Mahlzeiten in der Früh und zu Mittag.
Phase 2: Doppelte Konzentration von *CARICOL*^{®}*,* Tagesdosis 40 ml, das entspricht der Menge von 2x2 Esslöffel. Die Einnahme erfolgte jeweils kurz vor den Mahlzeiten in der Früh und zu Mittag.

### Dokumentation:

Von jedem teilnehmenden Bewohner wurden anamnestische Daten, wie Name, Alter, Geschlecht, Operationen, Diagnosen und laufende Medikation in einem speziellen Datenblatt aufgelistet.

Die tägliche Applikation von *CARICOL*^{®} sowie die Stuhlform wurden in einem sogenannten Stuhlblatt dokumentiert.

Es wurde in den Aufzeichnungen differenziert zwischen folgenden Stuhlformen:
0 = kein Stuhl,
I = normal geformt + = Stuhlspuren,
- = flüssig
§ - breiig
H = hart,

### Ergebnisse:

Zu Beginn der Vorlaufphase lag der durchschnittliche Anteil breiiger/flüssiger Stuhl bei 4 Tage/Woche, jener mit Normalstuhl bei 2,5 Tage/Woche.
Bereits kurz nach Beginn der Therapiephase konnte die Gleichheit zwischen breiiger/flüssiger Stuhl und Normalstuhl erreicht werden.
In der letzten Woche der Phase 1 erreichte der durchschnittliche Anteil mit Normalstuhl etwa 5 Tage/Woche und der durchschnittliche Anteil breiiger/flüssiger Stuhl konnte auf etwa 1 Tag pro Woche reduziert werden.

In diesem Beobachtungszeitraum der Phase 1 konnte bei allen 10 Probanden ein überwiegender Anteil an Tagen mit breiigen/flüssigen Stühlen in einen überwiegenden Anteil an Tagen mit Normalstuhl umgekehrt werden.

In der Phase 2 lag nach dreiwöchigem Absetzen zu Beginn der Therapie der durchschnittliche Anteil Normalstuhl nur noch bei etwa 3,8 Tage/Woche (nach 5 am Ende der ersten Phase), jener des breiig/flüssigen Stuhls bei 2,7 Tage/Woche.
In der letzten Woche der Phase 2 erreichte der durchschnittliche Anteil mit Normalstuhl etwa 3,1 Tage/Woche und der durchschnittliche Anteil breiiger/flüssiger Stuhl konnte auf etwa 1,8 Tag pro Woche reduziert werden.

### Der Vergleich zwischen Phase 1 und Phase 2 zeigt:

Bei geriatrischen Patienten tendiert nach Absetzen der Therapie der Stuhl wieder zur ursprünglichen Form.
Die Therapie in Phase 1 mit der einfachen *CARICOL*^{®}-Konzentration führt bei chronischer Diarrhoe zu besseren Ergebnissen als die Therapie mit der doppelten Konzentration in der Phase 2.
In der Phase 1 war die Zeit vom Therapiebeginn bis.zum Ansprechen wesentlich kürzer als in der Phase 2, und es erreichte der Normalstuhl in der Phase 1 wesentlich höhere Werte als in der Phase 2 (die Schere zwischen Normalstuhl und breiig/flüssigem Stuhl geht in Phase 1 wesentlich weiter auf als in der Phase 2,

Auch konnten im Beobachtungszeitraum der Phase 2 mit doppelter CARICOL-Konzentration nur mehr bei 8 von den 10 Probanden der überwiegende Anteil an Tagen mit breiigen/flüssigen Stühlen in einen überwiegenden Anteil an Tagen mit Normalstuhl umgekehrt werden.

### Zusammenfassung:

In dieser an 10 Patienten bzw. Bewohnern einer geriatrischen Abteilung (Multimorbidität, teilweise Immobilität) durchgeführten Anwendungsbeobachtung mit einem so genannten Nahrungsergänzungsmittel, einer erfindungsgemäß zubereiteten Form des Fruchtfleisches der Papaya (Caricol) konnte ein signifikanter Effekt auf die Verbesserung der Stuhlgewohnheiten bei chron. Diarrhoe gezeigt werden.
Zu erwähnen ist eine überraschende, bemerkenswert weitreichende Besserung eines seit Jahren bestehenden Ulcus cruris bei einem der beobachteten Patienten während der Einnahme.

### 6. Weitere Studie zur Wirksamkeit des erfindungsgemäßen Papaya-Präparates bei geriatrischen Patienten mit chronischer Obstipation:

### STUDIENDESIGN

In die Studie wurden 18 Patienten einer geriatrischen Abteilung, die großteils immobil sind, aufgenommen. Alle hatten die ärztliche Diagnose einer Obstipation und erhielten tgl. bis mehrmals wöchentlich Laxanzien.

Ausgeschlossen waren Patienten bzw. Bewohner, die ein oder mehrere der aufgeführten Kriterien erfüllten:
- Bekanntes Malignom
- Stoma
- Blut im Stuhl
- Höhergradige Herzinsuffizienz
- Vorliegende Morphintherapie

Der Studienablauf gliederte sich in eine Vorlaufperiode von 25 Tagen, eine Untersuchungsperiode 1 von 35 Tagen, in denen 2 x tgl. das erfindungsgemäße Präparat verabreicht wurde (3 EL vor dem Frühstück mit etwas Wasser, 3 EL vor dem Mittagessen mit etwas Wasser), eine Untersuchungsperiode 2 von 21-28 Tagen, in der das Präparat auf eine morgendliche Verabreichung reduziert wurde (2 EL vor dem Frühstück mit etwas Wasser).

Während des gesamten Untersuchungszeitraumes wurde das Stuhlverhalten der Patienten äußerst genau auf speziellen Dokumentationsblättern notiert.

Als "escape"-Therapie, also wenn trotz Verabreichung des Studienpräparates kein Stuhlgang erfolgte, wurden am 3.Tag ohne Stuhlgang 2 Lecicarbon® appliziert, am 4.Tag ohne Stuhlgang 2 Mikroklist®.

### STUDIENVERLAUF

Es wurden 18 Patienten in die Studie aufgenommen, die die oben genannten Kriterien erfüllten.

In der **Untersuchungsphase 1** zeigten 12 Patienten (von insgesamt 18) eine Verbesserung des Obstipationsgrades (Quotient aus Anzahl der Tage ohne Stuhl dividiert durch Anzahl der Tage mit Stuhl. 0 bedeutet täglicher Stuhlgang, 1 bedeutet 50% der Tage sind ohne Stuhlgang, etc.) im Vergleich zur Vorlaufphase. 2 Patienten zeigten eine geringe Zunahme der stuhlfreien Tage im Vergleich zur Vorlaufphase, zeigten jedoch in der Untersuchungsphase 2 eine deutliche Besserung im Vergleich zur Vorlaufphase. 4 Patienten zeigten eine Verschlechterung des Obstipationsgrades.
In der **Untersuchungsphase 2** wurden insgesamt noch 14 Patienten weiterbeobachtet. Von diesen zeigten 5 Patienten trotz Dosisreduktion eine weitere kontinuierliche Abnahme des Obstipationsquotienten. 3 Patienten zeigten trotz Reduktion der Dosis auf einmal 2 Eßlöffel vor dem Frühstück (Dosisdrittelung) in der Untersuchungsphase 2 keine Veränderung zur Untersuchungsphase 1. 3 Patienten zeigten in der Untersuchungsphase 2 (Dosisreduktion) eine Verschlechterung im Vergleich zu Untersuchungsphase 1, jedoch eine deutliche Verbesserung im Vergleich zur Vorlaufphase. Bei 3 Patienten kam es im Vergleich zur Vorlaufphase zu einem Anstieg des Obstipationsgrades. Hier wäre noch eigens eine Untersuchung mit höherer Dosierung des Studienpräparates empfehlenswert.

Bemerkenswert ist neben der Entwicklung des Obstipationsgrades der Verlauf der Interventionen. Musste während der ersten Woche der **Vorlaufphase noch 21** mal eine "escape medication" verabreicht werden, konnten die Interventionen in der letzten Woche der **untersuchungsphase 1 auf 9** und in den letzten Wochen der **Untersuchungsphase 2 (Dosisreduktion) bis auf 5 bzw.** 3 reduziert werden.

### ZUSAMMENFASSUNG

In dieser an 18 Patienten einer geriatrischen Abteilung (Immobilität, Multimorbidität) durchgeführten prospektiven Studie mit einem so genannten Nahrungsergänzungsmittel, einer erfindungsgemäß zubereiteten Form des Fruchtfleisches der Papaya (=CARICOL^{®}) konnte ein signifikanter Effekt auf die Verbesserung der Stuhlgewohnheiten ohne Intervention mit Laxanzien nachgewiesen werden.

### 7. Weitere klinische Beobachtungen im Rahmen der durchgeführten Versuche:

Im Rahmen der nach oben unter 3.-6. durchgeführten Studien wurden die folgenden klinischen Beobachtungen berichtet:

Ein Patient, der schon längere Zeit nicht mehr aufstehen wollte und geistig sehr abwesend war, bekam nach einigen Tagen der Einnahme des erfindungsgemäßen Mittels neue Energien, wollte wieder aus dem Bett und sein Sprache wurde wieder klarer.

Bei einer Frau, die seit über einem Jahr an einem offenen Geschwür am Fuß (Ulcus cruris) litt, führten verschiedene Behandlungen, inklusive Hauttransplantationen zu keinen Verbesserungen. Obwohl diese Frau keine Verdauungsprobleme hatte, wollte sie das erfindungsgemäße Mittel unbedingt nehmen und hat das auch über drei Monate sehr konsequent gemacht, mit dem Effekt, dass der Fuß verheilt ist.

Bei einer Diabetikerin wurde nach wenigen Wochen der Einnahme des erfindungsgemäßen Mittels ein offener Fuß wesentlich schöner und weniger Insulin musste gespritzt werden.

Bei einem unter Parkinson leidenden Patienten konnte nach einer Therapie mit dem erfindungsgemäßen hergestellten Papaya-Extrakt seine Motorik sehbar verbessert werden.

Aus diesen Beobachtungen im Rahmen der durchgeführten Studien zu den Indikationen "chronische Obstipation" und "chronische Diarrhoe" konnte daher entnommen werden, dass das erfindungsgemäße Mittel jedenfalls auch positive Auswirkungen auf den Stoffwechsel, die Abwehrkräfte, den Zustand von Parkinson Patienten, die Wundheilung, als auch auf die Durchblutung hat.

## Patentansprüche

1. Verwendung eines Mus-Präparates aus Carica papaya-Früchten, erhältlich nach einem Verfahren, **gekennzeichnet durch** die folgenden Schritte:
- Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
- Abkühlen der gekochten Früchte oder zerkleinerten Früchte über einen Zeitraum von zumindest 30 min unter Sauerstoff-haltiger Atmosphäre,
- gegebenenfalls Zerkleinern, Mischen und Passieren der abgekühlten Früchte oder zerkleinerten Früchte bis zum Erhalt eines homogenen Muses zur Herstellung eines Mittels zur Behandlung von Verdauungsstörungen.

2. Verwendung nach Anspruch 1, **dadurch gekennzeichnet, dass** das Kochen für mindestens 2 h durchgeführt wird.

3. Verwendung nach Anspruch 1 oder 2, **dadurch gekennzeichnet, dass** das Abkühlen für mindestens 5 h durchgeführt wird.

4. Verwendung nach einem der Ansprüche 1 bis 3, **dadurch gekennzeichnet, dass** bei der Herstellung des Muses Zitronensäure zugegeben wird, vorzugsweise in einer Menge, die zu einem pH-Wert des Muses auf 3,5 bis 5,0, insbesondere 3,8 bis 4,4, führt.

5. Verwendung nach einem der Ansprüche 1 bis 4, **dadurch gekennzeichnet, dass** die *Carica papaya*-Früchte vor dem Kochen geschält und entkernt werden.

6. Verwendung nach einem der Ansprüche 1 bis 5, **dadurch gekennzeichnet, dass** das erhaltene Mus pasteurisiert wird.

7. Verwendung nach einem der Ansprüche 1 bis 6, **dadurch gekennzeichnet, dass** die *Carica papaya*-Früchte halbreif bis reif sind.

8. Verwendung nach einem der Ansprüche 1 bis 7, **dadurch gekennzeichnet, dass** die Verdauungsstörung ausgewählt ist aus der Gruppe chronische Obstipation, Blähung und Reizdarmsyndrom.

## Claims

1. Use of a puree preparation from *Carica papaya* fruits obtainable with a method which is **characterized by** the steps of:
- cooking the fruits or crushed fruits, in particular fruits in sieved form, for at least 30 minutes at normal pressure, optionally with at least twice the volume of an aqueous solution,
- cooling the cooked fruits or crushed fruits for a period of at least 30 minutes in an oxygen-containing atmosphere,
- optionally crushing, mixing and straining the cooled fruits or crushed fruits until a homogenous puree is obtained, for the production of an agent for treating digestive disorders.

2. Use according to claim 1, **characterized in that** said cooking is carried out for at least 2 hours.

3. Use according to claim 1 or 2, **characterized in that** said cooling is carried out for at least 5 hours.

4. Use according to one of claims 1 to 3, **characterized in that** citric acid is added during preparation of the puree, preferably in an amount that results in a pH of the puree in the range 3.5 to 5.0, in particular 3.8 to 4.4.

5. Use according to one of claims 1 to 4, **characterized in that** the *Carica papaya* fruits are peeled and stoned prior to cooking.

6. Use according to one of claims 1 to 5, **characterized in that** the puree obtained is pasteurized.

7. Use according to one of claims 1 to 6, **characterized in that** the *Carica papaya* fruits are half-ripe to ripe.

8. Use according to one of claims 1 to 7, **characterized in that** the digestive disorder is selected from the group consisting of chronic constipation, flatulation and irritable bowel syndrome.

## Revendications

1. Utilisation d'une préparation à base de compote de fruits de *Carica papaya* qui peut être obtenue par un procédé comprenant les étapes suivantes :
- cuisson des fruits ou de morceaux de fruits, en particulier de fruits passés au crible, pendant au moins 30 minutes sous une pression normale, le cas échéant avec un volume au moins double d'une solution aqueuse,
- refroidissement des fruits ou morceaux de fruits cuits sur une durée minimale de 30 minutes sous une atmosphère contenant de l'oxygène,
- le cas échéant broyage, mélange et passage des fruits ou morceaux de fruits refroidis jusqu'à obtenir une compote homogène, afin de préparer un agent destiné au traitement de troubles digestifs.

2. Utilisation selon la revendication 1, **caractérisée en ce que** la cuisson dure au moins 2 heures.

3. Utilisation selon la revendication 1 ou la revendication 2, **caractérisée en ce que** le refroidissement dure au moins 5 heures.

4. Utilisation selon l'une des revendications 1 à 3, **caractérisée en ce que,** lors de la préparation de la compote, de l'acide citrique est ajouté, de préférence en une quantité qui permet d'ajuster le pH de la compote à une valeur allant de 3,5 à 5,0, en particulier de 3,8 à 4,4.

5. Utilisation selon l'une des revendications 1 à 4, **caractérisée en ce que** les fruits de *Carica papaya* sont épluchés et dénoyautés avant la cuisson.

6. Utilisation selon l'une des revendications 1 à 5, **caractérisée en ce que** la compote obtenue est soumise à une pasteurisation.

7. Utilisation selon l'une des revendications 1 à 6, **caractérisée en ce que** les fruits de *Carica papaya* sont demi-mûrs à mûrs.

8. Utilisation selon l'une des revendications 1 à 7, **caractérisée en ce que** le trouble digestif est choisi dans le groupe constitué par la constipation chronique, la flatulence et le syndrome de l'intestin irritable.
